# EUROPEAN PATENT SPECIFICATION

(11) **EP 0 771 528 B1**
(45) Date of publication and mention of the grant of the patent: **24.04.2002**
(21) Application number: 95307764.1
(22) Date of filing: 31.10.1995
(51) Int. Cl.: A01N 59/12

(54) **Bovine teat dip formulation**
Zusammensetzung zum Eintauchen von Rinder-Zitzen
Composition pour immerger les trayons de bovines

(43) Date of publication of application: 07.05.1997
(73) Proprietor: Devtech Marketing, Inc., Irvine, California 92714 (US)
(72) Inventor: Ricketts, David J., Irvine, California 92714 (US)
(74) Representative: West, Alan Harry

(56) References cited:
- US-A- 3 728 449
- US-A- 4 049 830

## Description

This invention relates to a bovine iodophor teat dip containing I₂ and I⁻ (or HI), and glycerine which is suitable for reducing the spread of mastitis infection, and improving teat appearance and skin condition; this in turn enables faster let down and milk out.

Many types of similar teat dip formulations have been employed in the past, and typical examples are found in U.S Patents 2,977,315; 3,950,554; 4,049,830; 4,258,056; 4,371,517; 4,671,958; 4,678,668; 4,940,702; 5,028,427; 5,175,160 and 5,208,257; and in German Patent 2,936,934.

The use of I₂ with I⁻ (or HI) which complexes (rather than reacts) with the surfactant to produce unwanted and possibly toxic reaction products, is described in U.S. Patents 2,599,140, 2,710,277 and 2,977,315 which use tamed iodine or iodophor. Also, the use of polyethenoxy detergents and I₂ is described in an article by Benjamin Carroll in the Journal of Bacteriology, 69; 413-417, (1955). In an attempt to avoid the use of I₂ and I⁻ (or HI) U.S. Patent 4,049,830 describes the use of bromo-nitro-propanol as the germicide in teat dips but this latter formulation is considered to have insufficient germicidal properties compared to iodine itself.

Another germicidal teat composition is described in U.S. Patent 3,728,449. That composition is an aqueous nonionic carrier-iodine formulation providing 0.25 to 2% available iodine and contains nonionic carriers selected from alkylphenol ethylene oxide condensates, long chain polyethers, ethoxylated partial esters of fatty acids with sugar alcohols such as sorbitol, butoxy derivatives of propylene oxide/ethylene oxide block copolymers, polyvinylpyrrolidone and mixtures thereof.

Present commercial teat dip formulations have been developed with the intent of combining desired germicidal properties with suitable emolliency, and include surfactants such as 9 to 12 mole ethyoxylated phenols containing I₂, I⁻ and glycerine. A surfactant of this type employed in a teat dip is sold by Norman Fox & Co. under the trade name NORFOX N-P9, and listed in "McCutcheon's Emulsifiers and Detergents", 1989 specifically for use with iodophors.

However, the NORFOX N-P9 formulation tends to cause the teat skin to become chapped, cracked and calloused, which in turn leads to infection, can provide a mastitis site, and may weaken the cow for other infections, delayed milk let down, and bovine discomfort when milking.

Another type of teat dip is sold as Klenzade™ Teat Guard, and contains a nonyl phenoxypolyethoxy ethanol surfactant having 1% titratable iodine, but this product does not keep the teat skin soft.

Still other bovine teat dip formulations have employed an emollient such as lanolin, but these formulations have failed to exhibit the desired characteristics of emolliency.

The ideal bovine teat dip formulation will have a suitable capability for dispersing I₂ and I⁻ (or HI) ; will not form toxic or objectionable compounds with I₂; will improve the teat appearance; will reduce teat callousing and cracking; will improve milk let down; will reduce discomfort when milking; will maintain a suitable capability for reducing the spread of mastitis; will have a reasonably good phase stable shelf life over a reasonably wide temperature range; and will be inexpensive to formulate.

The present invention now seeks to meet these objectives by providing an iodophor teat dip formulation comprising an emulsifier/dispersant of water soluble, nonionic, fatty alcohol polyglycol ether carboxylic acids; glycerine or the like; effective amounts of I₂ and I⁻ (or HI or the like) ; and a pH buffer.

According to the invention, therefore, there is provided a bovine teat dip formulation comprising an aqueous solution of
a non-ionic laureth (C11-17) carboxylic acid having an HLB of 10-16, in an amount of 7.5-12.5 weight %, based on the total formulation;
an iodophor;
glycerine or the like emollient in an amount of 5-12.5 weight %, based on the total formulation;
a buffer; and
an agent to adjust the pH of the formulation to 4.0-5.5.

A suitable emulsifier/dispersant for use in these formulations is sold by Alcolac Inc. under the trade names of AKYPO™ RLM-45, AKYPO™ RLM-100, AKYPO™ RLM-130 and AKYPO™ RLM- 160, the preferred composition being AKYPO™ RLM-100 (Chemical Abstracts Registry 74349-89-6), although mixtures of such compositions may be used.

These AKYPO™ RLM emulsifiers/dispersants are listed in "McCutcheon's Emulsifiers & Detergents", 1989 for personal care products to improve skin feel, in high electrolyte household and industrial formulae, and as emulsifiers for synthetic latex, but they are not listed for use in teat dip formulations.

However, according to the invention, it has been discovered unexpectedly, that when used in conjunction with appropriate concentrations of glycerine or the like, an iodophor such as I₂, I⁻ (or HI) and a pH buffer, these water-soluble emulsifiers/dispersants produce a teat dip having the requisite properties. An adequate, liquid phase stable life over a wide range of ambient temperatures is obtained, and moreover, the emulsifier/dispersant complexes the iodine and maintains the iodine in solution, thereby preventing the iodine from precipitating and forming a separate, solid phase. Also, the emulsifier/dispersants function together with the glycerine to impart the necessary emolliency for smooth teats, with reduced cracking, callousing and sores. In turn, this lessens the possibility of infection sites leading to mastitis, and other diseases.

The AKYPO™ RLM emulsifiers/dispersants used in the formulation have a range of laureth (11-17) carboxylic acids, with an HLB (Hydrophilic/Lipophilic Balance) number from about 10 to 16, and the AKYPO™ RLM-100 itself has an HLB of 14.8.

Typical formulations comprise I₂ in an amount of 0.45-1.3%, HI or its equivalent (for example KI, NaI, CaI₂ and all mixtures thereof) in an amount to form 0.25 to 0.3% I⁻; glycerine, or the like in an amount of 5-12.5%; a buffer such as citric acid and/or lactic acid; alkali for adjusting the pH to about 4.0-5.5, and preferably to about 4.3-5.2; the emulsifier/dispersant in an amount of 7.5-12.5%; and the balance of water.

If the glycerine content is below about 5%, the formulation does not appear effective. Other hydroxy equivalents to glycerine may be employed, and include sorbitol, mannitol, galacticol, 1,2,6-hexanetriol, non-ionic polyethylene glycols having molecular weights of 100 to 800 propylene and di-propylene glycols and pentitols such as arabitol and all possible mixtures thereof. However, glycerine is preferred.

If the emulsifier/dispersant content is below about 7.5%, the formulation becomes unstable due to lack of iodine complexing capability, and if the emulsifier/dispersant content is above about 12.5%, no significant improvement is observed.

Using an AKYPO™ RLM-100 emulsifier/dispersant in an amount of about 10%, an I₂ content of about 1.05%, an I⁻ content of about 0.28%, about 10% glycerine, citric acid to buffer the pH, alkali to obtain a pH range of about 4.8 - 5.2 and the balance water (all parts by weight), a suitable formulation may be produced having a phase-stable shelf life of about two years within an ambient temperature range of about -1 to 45°C.

The following Examples illustrate the invention.

### Example 1

Using the above formulation, several herds totalling 2,600 dairy cows were treated during a four months' period with about 625,000 teat dips. The herds initially had a low mastitis and infection rate, and use of the above formulation neither improved nor worsened the condition of the herds. However, there was a significant improvement in teat appearance, such as smoother teats, with significantly fewer cracks and as a consequence lessened opportunity for the spread of infectious disease such as mastitis. Also, there was noted a greater improvement in faster let down and onset of milking, indicating lessened irritation of the cow teats.

The life span of cows varies from about three to seven years, and during a prolonged period from the commencement of the four months tests, no significant change in the life span of these herds was observed.

### Example 2

12 Dairy herds of about 200 cows each were treated with the above formulation, and a noticeable improvement in teat condition was observed within about ten days. Few cracks, sores and callousing appeared, resulting in the cow teats being much easier to prepare for milking and as a consequence faster milking operation due to less discomfort.

Also, there was no increase in the somatic (white cell) count from solutions taken from the teat area, an increase in this cell count being the first sign of a reduced resistance to infectious disease such as mastitis. Additionally, there was a decreased reaction of milkers' hands such as chapping, compared to using the NORFOX NP-9 nonyl phenoxy surfactant.

The teat dip formulations of the invention provide good germicidal properties, and impart to cows a smoother teat with fewer callouses, roughness, sores and cracking. Use of the formulations thus reduces entry sites for infection and subsequent spreading of disease, such as mastitis, and milking is made easier, due to less teat irritation. Moreover, the teat dip formulations of the invention is relatively inexpensive and having a reasonably good shelf life at ambient conditions.

## Claims

1. A bovine teat dip formulation comprising an aqueous solution of
a non-ionic laureth (C11-17) carboxylic acid having an HLB of 10-16, in an amount of 7.5-12.5 weight %, based on the total formulation;
an iodophor;
glycerine or the like emollient in an amount of 5-12.5 weight %, based on the total formulation;
a buffer; and
an agent to adjust the pH of the formulation to 4.0-5.5.

2. A formulation according to claim 1, wherein the laureth (C11-17) carboxylic acid has an HLB of 14.8.

3. A formulation according to claim 1 or claim 2, wherein the iodophor comprises I₂ in an amount of 0.45-1.3 weight % based on the total formulation, and HI or its equivalent to provide an I⁻ content of 0.25-0.3 weight % based on the total formulation.

4. A formulation according to claim 3, wherein the iodophor comprises HI, KI, NaI or CaI₂ or a mixture of any two or more thereof.

5. A formulation according to any one of claims 1 to 4, wherein the buffer is selected from citric acid, lactic acid and mixtures thereof, and wherein the formulation has a pH of 4.8-5.2.

6. A formulation according to any one of claims 1 to 5, wherein the emollient is selected from glycerine, sorbitol, mannitol, galacticol, propylene and dipropylene glycols, 1,2,6-hexanetriol, pentitols including arabitol, polyethylene glycols having molecular weights of 100-800, and mixtures of any two or more thereof.

7. A formulation according to claim 1, comprising (based on the weight of the total formulation)
10% non-ionic laureth (C11-17) carboxylic acid;
0.25-0.3% I⁻;
10% glycerine;
a buffer;
alkali to a pH of 4.0-5.5; and
water.

8. A formulation according to claim 7, wherein the laureth (C11-17) carboxylic acid has an HLB of 14.8; the I⁻ content is about 0.28%; the buffer is citric acid or lactic acid; and the pH is 4.8-5.2.

9. A formulation according to any one of claims 1 to 8, having a phase stable shelf life of about two years at ambient temperature.

## Patentansprüche

1. Formulierung zum Eintauchen von Rinder-Zitzen, umfassend eine wässrige Lösung von:
einer Nonionic-Laureth-(C11-17)-carbonsäure mit einem HLB-Wert von 10 -16 in einer Menge von 7,5 bis 12,5 Gew.-%, bezogen auf die Gesamtformulierung,
einem Jodophor,
Glycerin oder einem ähnlichen geschmeidigmachendes Mittel in einer Menge von 5 - 12,5 Gew.-%, bezogen auf die Gesamtformulierung,
einem Puffer, und
einem Mittel zum Einstellen des pH der Formulierung auf 4,0 - 5,5.

2. Formulierung nach Anspruch 1, in welcher die Laureth-(C11-17)-carbonsäure einen HLB-Wert von 14,8 hat.

3. Formulierung nach Anspruch 1 oder Anspruch 2, in welcher der Jodophor I₂ in einer Menge von 0,45 - 1,3 Gew.-%, bezogen auf die Gesamtformulierung, und HI oder sein Äquivalent, um einen I⁻-Gehalt von 0,25 - 0,3 Gew.-%, bezogen auf die Gesamtformulierung bereitzustellen, umfasst.

4. Formulierung nach Anspruch 3, in welcher der Jodophor HI, KI, NaI oder CaI₂ oder eine Mischung von irgendwelchen zwei oder mehr hiervon umfasst.

5. Formulierung nach irgendeinem der Ansprüche 1 bis 4, in welcher der Puffer aus Zitronensäure, Milchsäure und Mischungen hiervon ausgewählt ist, und in welcher die Formulierung einen pH von 4,8 - 5,2 hat.

6. Formulierung nach irgendeinem der Ansprüche 1 bis 5, in welcher das geschmeidigmachende Mittel ausgewählt ist aus Glycerin, Sorbit, Mannit, Galactit, Propylen- und Dipropylenglykolen, 1,2,6-Hexantriol, Pentiten einschließlich Arabit, Polyethylenglykolen mit Molekulargewichten von 100 - 800 und Mischungen von irgendwelchen zwei oder mehreren hiervon.

7. Formulierung nach Anspruch 1, umfassend (bezogen auf das Gewicht der Gesamtformulierung)
10% Nonionic-Laureth-(C11-17)-carbonsäure,
0,25 - 0,3 % I⁻,
10% Glycerin,
einen Puffer,
Alkali bis zu einem pH von 4,0 - 5,5, und
Wasser.

8. Formulierung nach Anspruch 7, in welcher die Laureth-(C11-17)-carbonsäure einen HLB-Wert von 14,8 hat, der I⁻-Gehalt etwa 0,28% ist, der Puffer Zitronensäure oder Milchsäure ist und der pH 4,8 - 5,2 ist.

9. Formulierung nach irgendeinem der Ansprüche 1 bis 8, die eine Phasen-Lagerzeit von etwa zwei Jahren bei Umgebungstemperatur hat.

## Revendications

1. Composition pour immerger les trayons de bovins, comprenant une solution aqueuse qui comprend :
- un acide laureth (C₁₁₋₁₇-carboxylique) non ionique présentant une balance hydrophile-lipophile (ou HLB) de 10-16, en une quantité de 7,5 à 12,5 % en poids par rapport au poids total de la composition,
- un iodophore,
- la glycérine ou un émollient similaire en une quantité de 5 à 12,5 % en poids par rapport au poids total de la composition,
- un tampon, et
- un agent pour ajuster le pH de la composition à une valeur de 4,0-5,5.

2. Composition selon la revendication 1, dans laquelle l'acide laureth (C₁₁₋₁₇-carboxylique) présente une HLB de 14,8.

3. Composition selon la revendication 1 ou 2, dans laquelle l'iodophore comprend I₂ en une quantité de 0,45 à 1,3 % en poids par rapport au poids total de la composition, et HI ou son équivalent pour donner une teneur en I⁻ de 0,25 à 0,3 % en poids par rapport au poids total de la composition.

4. Composition selon la revendication 3, dans laquelle l'iodophore comprend HI, KI, NaI ou CaI₂ ou un mélange de deux quelconques de ces derniers ou plus.

5. Composition selon l'une queclonque des revendications 1 à 4, dans laquelle le tampon est choisi parmi l'acide citrique, l'acide lactique et leurs mélanges, et laquelle composition présente un pH de 4,8 à 5,2.

6. Composition selon l'une quelconque des revendications 1 à 5, dans laquelle l'émollient est choisi parmi la glycérine, le sorbitol, le mannitol, le galacticol, le propylène-glycol et les dipropylène-glycols, le 1,2,6-hexanetriol, les pentitols, y compris l'arabitol, les polyéthylèneglycols présentant des masses moléculaires de 100 à 800, et les mélanges de deux quelconques de ces derniers ou plus.

7. Composition selon la revendication 1, comprenant (par rapport au poids total de la composition) :
10 % d'acide laureth (C₁₁₋₁₇-carboxylique) non ionique,
0,25-0,3 % de I⁻,
10 % de glycérine,
un tampon,
un alcali permettant d'obtenir un pH de 4,0-5,5, et
de l'eau.

8. Composition selon la revendication 7, dans laquelle l'acide laureth (C₁₁₋₁₇-carboxylique) présente une HLB de 14,8 ; la teneur en I⁻ est d'environ 0,28 % ; le tampon est l'acide citrique ou l'acide lactique ; et le pH vaut 4,8-5,2.

9. Composition selon l'une quelconque des revendications 1 à 8, restant stable au niveau des phases pendant une durée de conservation en magasin d'environ 2 ans à température ambiante.
